# EUROPEAN PATENT SPECIFICATION

(11) **EP 0 366 385 B1**
(45) Date of publication and mention of the grant of the patent: **09.06.1993**
(21) Application number: 89310887.8
(22) Date of filing: 23.10.1989
(51) Int. Cl.: C07D 473/18, A61K 31/52

(54) **Guanine derivatives having antiviral activity and their pharmaceutically acceptable salts**
Guaninderivate mit antiviraler Wirkung und deren pharmazeutisch verträgliche Salze
Dérivés de guanine ayant une activité anti-virale et leurs sels pharmaceutiquement acceptables

(30) Priority: 24.10.1988 GB 8824899; 30.05.1989 GB 8912328
(43) Date of publication of application: 02.05.1990
(73) Proprietor: THE WELLCOME FOUNDATION LIMITED, London NW1 2BP (GB)
(72) Inventor: Daluge, Susan Mary, Chapel Hill North Carolina 27514 (US)
(74) Representative: Garrett, Michael

(56) References cited:
- EP-A- 0 236 935
- EP-A- 0 286 425
- WO-A-88/07532
- GB-A- 1 523 865
- US-A- 4 268 672
- CHEMICAL ABSTRACTS, vol. 110, no. 7, 13 February 1989, Columbus, OH (US); R. VINCE et al., pp. 15-16, no. 50717z#

## Description

This invention relates to amino acid esters of the carbocyclic purine nucleoside, (±)-9-(cis-4-(hydroxymethyl)-2-cyclopentenyl)guanine, and pharmaceutically acceptable salts thereof having valuable antiviral properties.

Acquired immunodeficiency syndrome (AIDS) is an immunosuppressive or immuno destructive disease that predisposes subjects to fatal opportunistic infections. Characteristically, AIDS is associated with a progressive depletion of T-Cells especially the helper-inducer subset bearing the OKT⁴ surface marker.

Human immunodeficiency virus (HIV) has been reproducibly isolated from patients with AIDS or with the symptoms that frequently precede AIDS. HIV is cytopathic and appears to preferentially infect and destroy T-Cells bearing the OKT⁴ marker. It is now generally recognised that HIV is the etiological agent of AIDS. Infection with HIV is also associated with clinical conditions such as AIDS-related complex (ARC), progressive generalised lymphadenopathy (PGL), AIDS-related neurological conditions, such as multiple sclerosis or tropical paraparesis, anti-HIV antibody-positive and HIV-positive conditions, such as thrombocytopenic purpura.

Since the discovery that HIV is the etiological agent of AIDS, numerous proposals have been made for anti-HIV chemotherapeutic agents that may be effective in treating AIDS sufferers. Thus, for example, certain nucleoside analogs and their use against HIV were described by Vince et al., Antiviral Research, 9(1/2), 120(1988). The carbocyclic purine nucleoside, (±)-9-(cis-4-(hydroxymethyl)-2-cyclopentenyl)guanine, (NSC-614846), also known as carbovir, and its anti-HIV activity were disclosed by Vince at the Second International Conference on Antiviral Research, Williamsburg, VA, 10-14 April, 1988.

Carbovir has also been found to have activity against the hepatitis B virus (HBV). HBV is a viral pathogen of world-wide major importance. The virus is aetiologically associated with primary hepatocellular carcinoma and is thought to cause 80% of the world's liver cancer. In the United States more than ten thousand people are hospitalised for HBV illness each year, an average of 250 die with fulminant disease. The United States currently contains an estimated pool of 500,000-1-million infectious carriers. Chronic active hepatitis generally develops in over 25% of carriers, and often progresses to cirrhosis.

Clinical effects of infection with HBV range from headache, fever, malaise, nausea, vomiting, anorexia and abdominal pains. Replication of the virus is usually controlled by the immune response, with a course of recovery lasting weeks or months in humans, but infection may be more severe leading to persistent chronic liver disease as outlined above.

We have now discovered that the amino acid esters of carbovir, which have not previously been disclosed in the literature, surprisingly have improved bioavailability after oral administration compared with carbovir itself.

According to the first feature of the present invention we provide the compounds of formula (I) 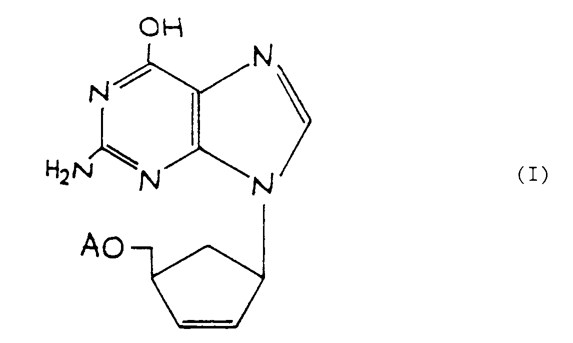
wherein A is the acyl residue of an aliphatic amino acid containing up to 7 carbon atoms and pharmaceutically acceptable salts thereof. The acyl residues may be in the D,L, or racemic DL configuration but preferably are in the L-form. The invention includes all antivirally active enantiomeric forms of the compounds of formula (I) either individually or admixed in any proportions.

Examples of the above amino acid acyl residues include those derived from naturally occuring amino acids, especially neutral amino acids, such as valine, isoleucine., threonine or t-butylglycine.

A particularly preferred amino acid ester is (1S, 4R)-[4-(2-amino-1,6-dihydro -6-oxo-9H-purin-9-yl)-2-cyclopenten-1-yl]-methyl-L-valinate.

In tests in rats, measuring the urinary recovery as carbovir (% dose administered) after oral administration, a compound of formula (I) above shows a large increase in absorption from the gut compared with carbovir. This enables less drug to be administered whilst still providing equivalent drug levels in the plasma after oral absorption.

In addition to the relatively high bioavailability, the amino acid esters of carbovir possess substantially the same antiviral effect in vitro as carbovir. The advantageous increase in bioavailability of the compounds is thus not gained at the expense of antiviral potency.

Pharmaceutically acceptable salts of a compound of formula (I) are preferably acid addition salts derived from an appropriate acid, e.g. hydrochloric, sulphonic, phosphoric, maleic, fumaric, citric, tartaric, lactic, acetic, trifluoroacetic or p-toluenesulphonic acid. Particularly preferred salts are the hydrochloride and trifluoroacetate salts of a compound of formula (I).

The present invention further provides:-
a) the compounds of formula (I) and pharmaceutically acceptable salts thereof for use in medical therapy, e.g. in the treatment or prophylaxis of a viral disease in an animal e.g. a mammal such as man.
b) the use of the compounds of formula (I) and pharmaceutically acceptable salts thereof in the manufacture of a medicament for the treatment or prophylaxis of a viral disease in an animal e.g. a mammal such as man.
c) a method for the treatment or prophylaxis of a viral disease in an animal e.g a mammal such as man which comprises administering to the said animal an effective antiviral amount of a compound of formula (I) or a pharmaceutically acceptable salt thereof.
d) a method of generating carbovir in vivo in an animal which comprises administering to the said animal a compound of formula (I) or a pharmaceutically acceptable salt thereof.

Examples of viral diseases (including viral infections and associated clinical conditions) which may be treated or prevented in accordance with the invention include human retroviral infections such as human immunodeficiency virus (HIV), and human T-cell lymphotropic virus (HLTV) e.g. HTLV-I or HTLV-II infections. The compounds of formula (I) and their salts are especially useful for the treatment or prophylaxis of AIDS and related clinical conditions such as AIDS-related complex (ARC), progressive generalized lymphadenopathy (PGL), AIDS-related neurological conditions such as multiple sclerosis or tropical paraparesis, anti-HIV antibody-positive and HIV-positive patients and HIV-related conditions such as thrombocytopenic purpura. The compounds may also be used in the treatment or prevention of psoriasis.

The compounds of the present invention have been found to be particularly applicable to the treatment of asymptomatic infections or diseases in humans caused by or associated with retroviruses.

The compounds of the present invention may also be used in the treatment or prophylaxis of hepatitis B virus infections and associated clinical disorders.

The above active ingredients (namely a compound of formula (I) or a pharmaceutically acceptable salt thereof) may be administered by any route appropriate to the condition to be treated, suitable routes including oral, rectal, nasal, topical (including buccal and sublingual) vaginal and parenteral (including subcutaneous, intramuscular, intravenous, intradermal, intrathecal and epidural). It will be appreciated that the preferred route may vary for example with the condition of the recipient.

For each of the above-indicated utilities and indications the amount required of an active ingredient (as above defined) will depend upon a number of factors including the severity of the condition to be treated and the identity of the recipient and will ultimately be at the discretion of the attendant physician or veterinarian. In general however, for each of these utilities and indications, a suitable effective dose will be in the range 0.1 to 250 mg per kilogram bodyweight of recipient per day, preferably in the range 1 to 100 mg per kilogram bodyweight per day and most preferably in the range 5 to 20 mg per kilogram bodyweight per day; an optimum dose is about 10 mg per kilogram bodyweight per day. (Unless otherwise indicated, all weights of active ingredient are calculated as a compound of formula (I): for salts thereof the figures would be increased proportionately.) The desired dose is preferably presented as two, three, four or more sub-doses administered at appropriate intervals throughout the day. These sub-doses may be administered in unit dosage forms, for example, containing 10 to 1000 mg, preferably 20 to 500 mg and most preferably 100 to 400 mg of active ingredient per unit dosage form.

The compounds of formula (I) and their pharmaceutically acceptable derivatives may be employed in combination with other therapeutic agents for the treatment or prophylaxis of the above infections or conditions. Examples of such further therapeutic agents include agents that are effective for the treatment or prophylaxis of HIV infections or associated conditions such as 3′-azido-3′-deoxythymidine (zidovudine), 2′,3′-dideoxynucleosides such as 2′,3′-dideoxycytidine, 2′,3′-dideoxyadenosine and 2′,3′-dideoxyinosine, and the 2′,3′-dideoxynucleosides described in European Patent Specification 286425, 2′,3′-dideoxydidehydronucleosides such as 2′,3′-deoxydidehydrothymidine (d4T), acyclic nucleosides (e.g. acyclovir), interferons such as α-interferon, renal excretion inhibitors such as probenicid, nucleoside transport inhibitors such as dipyridamole, as well as immunodulators such as interleukin II and granulocyte macrophage colony stimulating factors. The component compounds of such combination therapy may be administered simultaneously, in either separate or combined formulations, or at different times, e.g. sequentially such that a combined effect is achieved.

While it is possible for the active ingredients to be administered alone, it is preferable to present them as pharmaceutical formulations. The formulations, both for veterinary and for human use, of the present invention comprise at least one active ingredient, as above defined, together with one or more pharmaceutical carriers therefor and optionally other therapeutic ingredients. The carrier(s) must be acceptable in the sense of being compatible with the other ingredients of the formulation and not deleterious to the recipient thereof.

The formulations include those suitable for oral, rectal, nasal, topical (including buccal and sublingual), vaginal or parenteral (including subcutanous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers of finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For infections of the eye or other external tissues e.g. mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either paraffinic or a water-miscible ointment base. Alternatively, the active ingredients may be formulated in a cream with an oil-in-water cream base. In addition topical applications may be made transdermally by means of an iontophoretic device.

If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol and mixtures thereof. The topical formulations may desirably include a compound which enhances absorption or penetration of the active ingredient through the skin or other affected areas. Examples of such dermal penetration enhancers include dimethylsulphoxide and related analogues.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured basis, usually sucrose and acacia or tragacanth; pastilles comprising the active ingredient in an inert basis such as gelatin and glycerine, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described. Formulations for intramuscular administration are particularly preferred.

Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The present invention further provides veterinary compositions comprising at least one active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

For oral administration the compositions can be in the form of a tablet, granule drench, paste, cachet, capsule or feed supplement. Granules may be made by the well known techniques of wet granulation, precompression or slugging. They can be administered to animals in an inert liquid vehicle so as to form a drench, or in a suspension with water or oil base. Preferably further accessory ingredients such as a dispensing agent are included. These formulations preferably contain from 15 to 85% of the active ingredient.

According to a further feature of the present invention we provide a process for the preparation of a compound of formula (I) above and pharmaceutically acceptable salts thereof which comprises either :
a) reacting a compound of formula (II) 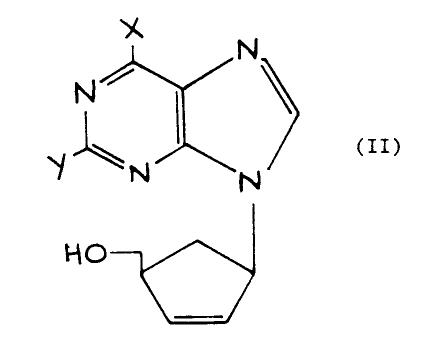 wherein X is an optionally protected hydroxy group, and Y is an optionally protected amino group, with an optionally protected aliphatic amino acid containing up to 7 carbon atoms or a functional equivalent thereof to introduce the acyl residue of an amino acid at the 5'-position; or
b) converting a compound of formula (III) 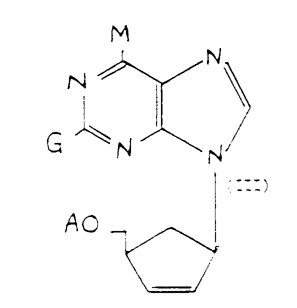 wherein A is as hereinbefore defined; and M represents a hydroxy group and G represents an atom or group that can be replaced by or converted to an amino group; or G represents an amino group and M represents an atom or group that can be replaced by or converted to a hydroxy group, into a compound of formula (I) or a pharmaceutically acceptable derivative thereof; or
c) reacting a compound of formula (IV) 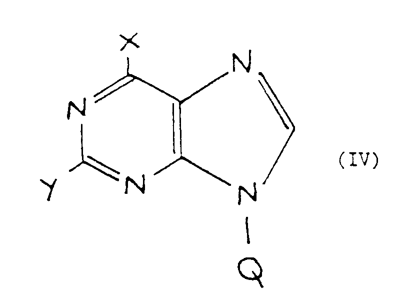 (wherein X and Y are as defined above and Q represents a leaving atom or group) with a compound of formula (V) 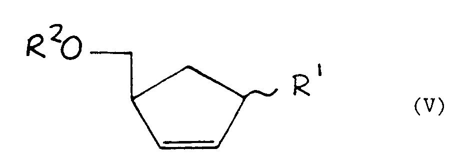 wherein R¹ represents a leaving group or atom and R² represents an optionally protected acyl residue of an amino acid; and optionally effecting one or more of the following conversions in any desired sequence :-
   i) removal of any protecting groups;
   ii) where the resulting product is a compound of formula (I), conversion of the said compound into a pharmaceutically acceptable salt thereof; and
   iii) where the resulting product is a pharmaceutically acceptable salt of a compound of formula (I), conversion of the said salt into the parent compound.

With regard to process a), the esterification reaction may be carried out in conventional manner, for example in a solvent such as pyridine or dimethylformamide in the presence of a coupling agent such as N,N′-dicyclohexylcarbodiimide, optionally in the presence of a catalytic base such as 4-dimethylaminopyridine. The water formed during the reaction may, if desired, be removed in conventional manner, for example by distillation or by the addition of a water-binding substance. Subsequently, the ester obtained as reaction product may be isolated in conventional manner.

As an alternative to the use of an amino acid per se, a functional equivalent of the acid may be employed, e.g. an acid halide such as the acid chloride, or an acid anhydride. In such a case in order to avoid undesirable side-reactions, it is advantageous to use an amino-protected derivative. Examples of preferred amino-protecting groups including acyl, e.g. C₁₋₄alkanoyl such as acetyl and aryloxycarbonyl, e.g. t-butyloxycarbonyl. A suitable amino- protected derivative, for example, is one wherein the amino group of the amino acid is replaced by an azido group.

Conversion of a compound of formula (III) into a compound of formula (I), by method b), can be achieved by various means. For example, G may represent a halogen atom or an alkylthio or alkylsulphonyl group which can be converted to an azide group which in turn can be converted to an amino group by reduction using, for example, triphenylphosphine and ammonium formate. For the preparation of the compound of formula (I), a compound of formula (III) wherein M is an amino group may be converted to a hydroxy group for example by treatment with a deaminating enzyme such as adenosine deaminase.

These processes together with other conventional processes are described in Fused Pyrimidines, Part II, Purines, Ed. by D.J.Brown (1971), Wiley-Interscience.

In process (c), the group Q in formula (IV) may, for example, represent a hydrogen atom; an acyl group, e.g. a C₁₋₄alkanoyl group such as an acetyl group or an aroyl group such an a benzoyl group; or a tri-C₁₋₄alkylsilyl group such as a trimethylsilyl group. The group R¹ in formula (V) may, for example, represent a halogen atom (e.g. chlorine) or an acyloxy group wherein the acyl moiety may be, for example, a C₁₋₄alkanoyl group such as acetyl or an aroyl group such as benzoyl. The amino group of the amino acid residue may be protected by for example, C₁₋₄alkanoyl (e.g. acetyl) or aryloxycarbanoyl (e.g. benzyloxycarbonyl); it may also represent an azido group. The reaction may be conveniently effected in a strong polar solvent such as dimethylformamide or hexamethylphosphoramide, advantageously in the presence of a base such as triethylamine or potassium carbonate. Alternatively, a thermal condensation may be effected by heating the compounds of formulae (IV) and (V) in the presence of a catalytic amount of a strong acid, e.g. sulphuric acid.

Compounds of formulae (II) to (V), employed as intermediates in the synthesis of a compound of formula (I), can be prepared in conventional manner, e.g. by procedures described in U.K. Patent Specification No. 1523865. These methods rely on intermediates prepared from simply substituted purines, which may be available commercially, or prepared according to techniques which are well known per se and which are disclosed in the literature such as the aforementioned text-book. Thus, for example, compounds of formula (III) may be generally prepared by using an analogous procedure to that of process (c), i.e. reacting an appropriate purine with a compound of formula (V).

The optional conversions i), ii) and iii) may be effected in conventional manner. Thus, for example, removal of protecting groups in conversion i) may be effected by hydrolysis or solvolysis as appropriate. With regard to removal of protecting groups on the amino acyl radicals, hydrolysis or solvolysis, eg. of alkyloxy or aryloxycarbonyl protecting groups are preferred. With regard to protection of the groups in the 2- and/or 6-position of the purine nucleus, these may be selected for example from alkyl groups (eg. 6-O-methyl may be removed with trimethylsilyl iodide in acetonitrile) or tri-C₁₋₄ alkylsilyl eg. trimethylsilyl (removable by solvolysis eg. by alcoholysis).

The conversion of a compound of formula (I) into a pharmaceutically acceptable salt may be effected in conventional manner, for example, by treatment of the compound with an appropriate acid to form an acid addition salt, for example, by lyophilisation of a methanolic solution of the parent ester with an acid solution.

Similarly, conversion of a salt into a compound of formula (I) may be effected in conventional manner.

The following Examples illustrate the present invention.

### Example 1

### Preparation of (1S, 4R)-[4-(2-Amino-1,6-dihydro-6-oxo-9H purin-9-yl)-2-cyclopenten-1-yl]-methyl-L-valinate trifluoroacetate

### A. (±)4-(1α,4α)-[(2-Amino-4-chloro-6-pyrimidinyl)amino]-2-cyclopentene-1-methanol

cis-4-Acetamidocyclopent-2-enemethyl acetate [Daluge and Vince, J.Org.Chem., 43, 2311 (1978) and U.S. Patent 4,268,672] (14.88g, 0.073 mole) and barium hydroxide octahydrate (46.19g, 0.146 mole) were refluxed in water (300 ml) under nitrogen for 18 hours. The resulting solution was neutralised with carbon dioxide. The precipitate was washed with water, then ethanol. The combined filtrate-wash was evaporated to a syrup (11.16g) which was condensed with 2-amino-4,6-dichloropyrimidine (23.91g, 0.146 mole) and triethylamine (30.5ml, 0.219 mole) in refluxing 1-butanol (100ml) for 1.5 hours. After addition of 1N NaOH (73ml), the resulting mixture was evaporated to dryness and the residual solid slurried in CHCl₃ (200ml). Unreacted 2-amino-4,6-dichloropyrimidine was filtered off and washed with chloroform (100ml). The chloroform filtrate-wash was concentrated and chromatographed on a silica gel column. Additional pyrimidine starting material was eluted with 2.5% methanol-chloroform. The title compound was eluted with 3.5% methanol-chloroform as an off-white solid foam (15.90g, 91%); ′H-NMR (Me₂SO-d6) δ 1.15-1.28 and 2.26-2.41 (2m, 2, CH₂), 2.60-2.71 (m, 1, 1′-H), 3.4 (m, overlapping H₂O, CH₂OH), 4.625 (t, J=5.3, 1, CH₂OH), 4.95 (br s, 1, CH-N), 5.67-5.87 (m, 3, CH=CH and pyrimidine H-5), 6.38 (br s, 1, NH₂), 7.12 (br s, 1, NH); MS (CI) M+1 : 241,243.

| | | | | |
|---|---|---|---|---|
| Anal. Calc. C₁₀H₁₃N₄OCl.0.2H₂O : | C,48.99; | H,5.55; | N,22.85; | Cl,14.46. |
| Found : | C,49.10; | H,5.57; | N,22.81; | Cl,14.40. |

### B. (±)-(1α,4α)-4-[[2-Amino-6-chloro-5-[(4-chlorophenyl)azo]-4-pyrimidinyl]amino]-2-cyclopentene-1-methanol

(±)4-(1α,4α)-[(2-Amino-4-chloro-6-pyrimidinyl)amino]-2-cyclopentene-1-methanol (11.58g, 48.1 mmole) and sodium acetate trihydrate (97g) were dissolved in glacial acetic acid (225ml) and water 225ml). A cold solution (0-5°C) of 4-chlorobenzenediazonium chloride was prepared from 4-chloroaniline (6.74g, 52.8 mmole), concentrated hydrochloric acid (14.7ml), water (52ml), and sodium nitrite (4.01g, 58.2mmol in 47ml of water). This cold solution was added dropwise over 5 minutes to the first solution. The resulting yellow precipitate was filtered after 18 hours, washed with water, and extracted with ethanol to give the title compound as a dark yellow powder (12.56g, 69%), mp 218-220°C dec : ′H-NMR (Me₂SO-d6) δ 10.25 (d, 1, NH), 7.69 and 7.54 (both d, J=8.9,) overlapping 7.6 (br, 6, C₆H₄ and NH₂), 5.80-5.95 (m, 2, CH=CH), 5.24 (m, 1, CHN), 4.75 (t, 1, CH₂OH), 3.41 (t, 2, CH₂OH), 2.75 (m, 1, CH), 2.41 (m, 1, CH), 1.44-1.53 (m, 1, CH).

| | | | | |
|---|---|---|---|---|
| Anal. Calc. C₁₆H₁₆N₆Cl₂O : | C,50.67; | H,4.25; | N,22.16; | Cl,18.70. |
| Found : | C,50.59; | H,4.29; | N,22.10; | Cl,18.66. |

### C. (±)-(1α, 4α)-[(2,5-Diamino-4-chloro-6-pyrimidinyl)-amino]-2-cyclopentene-1-methanol

The title compound of Example 1B (11.67g) was suspended in ethanol (235ml), glacial acetic acid (30ml), and water (235ml). The mixture was heated to reflux under nitrogen. Zinc dust (13.5g) was added in small portions over 30 minutes during which time the compound dissolved. The reaction was heated for an additional 20 minutes. and then the excess zinc was filtered off from the hot solution, and was washed with ethanol. The filtrates were evaporated, and the residue was purified on a silica gel column eluting with chloroform (1L) and chloroform:methanol/4:1 (1.8L). The fractions containing the product were combined, and the solvent was removed under reduced pressure to give the title compound as a red-orange oil (11.2g, > 100% yield). A pure sample was obtained during another small scale reaction to obtain the product as a light yellow solid in a 76% yield; ¹H-NMR (Me₂SO-d6) δ 1.29 and 2.39 (m, 2, CH₂), 2.69 (t, 1, 1′-H), 3.37 (d, 2, CH₂OH), 3.91 (br, 2, NH₂), 4.60 (br, 1, CH₂OH), 5.02 (m, 1, CHNH), 5.56 (br s, 2, NH₂), 5.74 (m, 1, = CH), 5.86 (m, 1, = CH), 6.36 (d, 1, CHNH).

### D. (±)-(1α,4α)-4-(2-Amino-6-chloro-9H-purin-9-yl)-2-cyclopentene-1-methanol

The title compound of Example 1C ( 9.7g) was dissolved in diethoxymethyl acetate (100g), and refluxed for two days. The solvent was removed under high vacuum at 50°C, and dioxane (40ml) and 0.5 N HCl (60ml) was added. The reaction was stirred at room temperature for 1.25 hours, and then chilled. The reaction was neutralized to pH 7 with cold 5 N sodium hydroxide, and then it was extracted with chloroform:methanol/3:1 several times. The organic layers were dried with magnesium sulfate, filtered, and evaporated. The residue was purified by chromatography on a silica gel column, eluting with 2% MeOH-CHCl₃ to give 3.7g (46% yield) of the title compound, mp 138-139°C : ¹H-NMR (Me₂SO-d₆) δ 1.63 and 2.61 (m, 2, CH₂), 2.87 (m, 1, 1′-H), 3.44 (m, 2, CH₂OH), 5.44 (m, 1, CH-N), 5.89 (m, 1, =CH), 6.14 (m, 1, = CH), 6.82 (br s, 2, NH₂), 8.02 (s, 1, 8-H). UV : pH 1 λmax 315 (ε 7370), 218 (26200); λsh 239.5 (5650); pH 7.4 λmax 307 (ε 8000), 245.5 (4600), 223 (26400). MS : (EI) 265,267 (M+); (CI) 266,268 (M+1).

| | | | | |
|---|---|---|---|---|
| Anal. Calc. C₁₁H₁₂N₅Cl0.2H₂O : | C,43.79; | H,5.35; | N,23.21; | Cl,11.75. |
| Found : | C,43.67; | H,5.29; | N,23.05; | Cl,11.70. |

### E. (±)-cis-4-(2,6-Diamino-9H-purin-9-yl)-2-cyclopentene-1-methanol

(±)-(1α,4α)-4-(2-Amino-6-chloro-9H-purin-9-yl)-2-cyclopentene-1-methanol (1.50g, 5.65mmol) and ammonia (1, 35ml) were stirred at 70°C in a Parr bomb for 48 hours. Evaporation left crude product which was purified by elution from a silica gel column with 10% methanol-chloroform (1.27g, 91%). Crystallisation of such a sample from acetonitrile-methanol gave white granules mp 145-147°C; ¹H-NMR (Me₂SO-d₆) δ 7.59 (s, 1, purine H-8) 6.60 (br s, 2, NH₂), 6.09 and 5.85 (both m, 2, CH=CH), 5.71 (br s, 2, NH₂), 5.36 (m, 1, CH-N), 4.71 (t, J=5.4, 1, OH), 3.50-3.35 (m, 2, CH₂-O), 2.85 (m, 1, H-1′), 2.60 and 1.57 (both m, 2, CH₂).

| | | | |
|---|---|---|---|
| Anal. Calc. C₁₁H₁₄N₆O : | C,53.65; | H,5.73; | N,34.13. |
| Found : | C,53.79; | H,5.79; | N,34.00. |

### F. (1R, 4S)-9-(4-Hydroxymethyl-2-cyclopentenyl)guanine

(±)-Cis-4-(2,6,diamino-9H-purin-9-yl)-2-cyclopentene-1-methanol (1.26g, 5.12 mmol) was dissolved in 514mL of 0.02M potassium phosphate buffer (pH 7.4) with 16.4x10³ units of calf intestinal adenosine deaminase (adenosine aminohydrolase EC3.5.4.4, Boehringer Mannheim). After 6.5 hours at 25°C, the reaction was quenched by addition of an equal volume of methanol. The solution was evaporated to dryness and the residual solids extracted with hot methane (4x100 ml). The contents of the methanol extracts was adsorbed on silica gel and loaded on a silica gel column packed in 10% MeOH-CHCl₃. Elution with 10% MeOH-CHCl₃ (400ml) gave unreacted starting material (0.66g). Crystallisation from methanol-acetonitrile gave white granules of (+)-cis-4-(2,6-diamino-9H-purine-9-yl)-2-cyclopentene-1- methanol (0.38g); mp 175-178°C; ¹H-NMR (Me₂SO-d₆) identical with that of starting material; [α]²⁰ + 85.1° (c=0.19 in methane). Continued elution of the column with 20-25% methanol chloroform (500mL) gave, on evaporation, a white powder (0.62g). Recrystallisation from water gave title compound as white needles (0.56g, 84%); mp>320°C; ¹H-NMR (Me₂SO-d₆) δ 10.54 (br s, 1, NHC=O), 7.59 (s, 1, H-8), 6.43 (br s, 2, NH₂), 6.1 and 5.8 (both m, 2, CH=CH), 5.3 (m, 1, CH-N), 4.73 (t, J=5.3, 1, OH), 3.43 (t, J=5.6, 2, CH₂-O), 2.8-2.6 (m, 1, H-4′), 2.65-2.55 and 1.60-1.50 (both m, 2, CH₂); [α]²⁰ - 96.3° (c=0.11 in .01N NaOH : MeOH/2:3); [α]²⁵ - 63.9°, [α]²⁵ - 140°C (c = 0.16, MeOH).

| | | | |
|---|---|---|---|
| Anal. Calc. C₁₁H₁₃N₅O₂.0.7H₂O : | C,50.84; | H,5.59; | N,26.95. |
| Found : | C,50.90; | H,5.63; | N,26.83. |

### G. (1S, 4R)-[4-(2-Amino-1,6-dihydro-6-oxo-9H-purin-9-yl)-2-cyclopenten-1-yl]-methyl-L-valinate trifluoroacetate

N-Butyloxycarbonyl-L-valine (197mg, 0.908 mmole) and N,N-dicyclohexylcarbodiimide (187mg, 0.908 mmole) were stirred in dry methylene chloride (8ml) for 30 minutes. The mixture was filtered, the precipitated urea washed with methylene chloride (3mL) and the filtrate wash evaporated to dryness. To the residual white solid (anhydride) was added (1R, 4S)-9-(4-hydroxymethyl-2-cyclopentenyl)guanine (106mg, 0.429mmole), dry N,N-dimethylformamide (4ml) and 4-dimethylaminopyridine (5.0mg, 0.04 mmole). The solution was stirred at 25°C under nitrogen for 48 hours, evaporated to dryness, and the residue chromatographed on silica gel. Elution with 10% methanol-chloroform gave the N-butyloxy-carbonyl-blocked derivative of title compound as white solid (188mg, 98%). ¹H-NMR (Me₂SO-d₆) δ 10.56 (br s, 1, NHCO), 7.60 (s, 1, H-8 of purine), 7.17 (d, 1, CHNHC=O), 6.42 (br s, 2, NH₂), 6.08 and 5.96 (both m, 2, CH=CH), 5.39 (m, 1, CH-N), 4.11 (d, 2, CH₂-OCO), 3.82 (t, 1, CH-N of valyl), 3.07 (m, 1, H-1 of cyclopentene), 2.63 (m, 1, 0.5 CH₂), 1.98 (m, 1 CHMe₂), 1.61 (m, 1, 0.5CH₂), 1.37 (s, 9, CMe₃), 0.87-0.85 (overlapping d, 6, Me₂CH). This derivative (183mg, 0.387 mmol) was dissolved in trifluoroacetic acid : methylene chloride/ 1:3 (13.5mL) and the solution stirred at 25°C for 45 minutes. Evaporation left title compound as white powder (182mg); foams at 120°C, mp 175-180°C; ¹H-NMR (Me₂SO-d₆) δ 10.76 (br s, 1, NHC=O) 8.33 (br s, 2.3, NH, NH₂⁺), 7.80 (s, 1, purine H-8), 6.56 (br s, 2, purine NH₂), 6.15-5.99 (m, 2, CH=CH), 5.40 (m, 1, H-4 of cyclopentene), 4.25 (d, J=6.3, 2, CH₂-O), 4.2-3.5 (m, 5, NH₃+, NCHC=O and partially exchanged NHD, HDO), 3.15 (m, 1, H-1 of cyclopentene), 2.8-2.6 (m, 1, 0.5CH₂), 2.2-2.05 (m, 1, CHMe₂), 1.7-1.6 (m, 1, 0.5CH₂), 0.99-0.93 (m, 6, CHMe₂).

| | | | |
|---|---|---|---|
| Anal. Calc. C₁₆H₂₂N₆O₃.1.8CF₃CO₂H : | C,42.68; | H,4.35; | N,15.23. |
| Found : | C,42.79; | H,4.59; | N,14.99. |

### H. (IS,4R)-[4-(2-Amino-1,6-dihydro-6-oxo-9H-purin-9-yl)-2-cyclopenten-1-yl]-methyl L-isoleucinate trifluoroacetate.

The title compound was prepared from N-butyloxycarbonyl-L-isoleucine and (1R,4S)-9-(4-hydroxymethyl-2-cyclopentenyl)guanine (130mg, 0.500mmol) in accordance with the procedures described in Example 1.G. Evaporation of the methylene chloride-trifluoroacetic acid left white solid foam (168mg, 55%), ¹H-NMR (DMSO-d₆)δ:10.82(s,1,NHCO), 8.33(br s,3,NH⁺), 7.88(s,1,H-8), 6.60(br s,2,purine NH₂) 6.15 and 6.0 (both m,2,CH=CH), 5.40(m,1,H-4 of cyclopentene), 4.24(d,J=6.3,2,CH₂-O), 4.0(br,m,1,N-CH-CO), 3.10(br,m,1,H-1 of cyclopentene), 2.80-2.60 (m,1,0.5CH₂), 1.95-1.8(b rm,1,CH(Me)Et), 1.70-1.60(m,1,0.5CH₂), 1.50-1.20(m,2,CH₂CH₃), 1.0-0.80(m,6,2CH₃); MS(CI):361(M+1).

| | | | |
|---|---|---|---|
| Anal. Calc. C₁₇H₂₄N₆O₃^{·}0.15H₂O ^{·} 2.15CF₃CO₂H : | C,42.06; | H,4.38; | N,13.82. |
| Found : | C,42.55; | H,4.60; | N,13.33. |

### I. (1S,4R)-[4-(2-Amino-1,6-dihydro-6-oxo-9H-purin-9-yl)-2-cyclopenten-1-yl)]-methyl(2S)-2-amino-3,3-dimethylbutyrate trifluoroacetate.

The title compound was prepared from N-butyloxycarbonyl-L-tert-leucine and (IR, 4S)-9-(4-hydroxymethyl-2-cyclopentenyl)guanine (130mg, 0.500mmol) in accordance with the procedures described in Example 1.G. Evaporation of the methylene chloride-trifluoroacetic acid left white solid foam (171mg, 52%); ¹H-NMR (DMSO-d₆)δ : 10.94(m,1,NHCO), 8.2-8.0(m,NH⁺) overlapping 8.00(s,purine H-8), 6.70(br s,2,purine NH₂) 6.15 and 5.95(both m,2,CH=CH), 5.40( m,1,H-4 of cyclopentene), 4.10-4.00(m,2,CH₂-O), 3.80(m,1,N-CH-CO), 3.20-3.00(brm,1,H-1 of cyclopentene), 2.80-2.60(m,1,0.5CH₂), 1.70-1.60(m,1,0.5CH₂), 1.1-0.8(m,9,3CH₃); MS(CI); 358(M+1).

| | | | |
|---|---|---|---|
| Anal. Calc. for C₁₇H₂₄N₆O₃^{·}0.2H₂O^{·}2.6CF₃CO₂H: | C,40.37; | H,4.12; | N,12.72. |
| Found : | C,41.39; | H,4.58; | N,11.67. |

### J. (1S,4R)-[4-(2-Amino-1,6-dihydro-6-oxo-9H-purin-9-yl)-2-cyclopenten-1-yl]-methyl L-threoninate trifluoroacetate.

The title compound was prepared from N-butyloxycarbonyl-L-threonine and (1R,4S)-9-(4-hydroxymethyl-2-cyclopentenyl)guanine (130mg, 0.500mmol) in accordance with the procedures described in Example 1.G. Evaporation of the methylene chloride-trifluoroacetic acid left white solid foam (215mg, 62%) ; ¹H-NMR (DMSO-d₆)δ : 10.87(brs,1,NHCO), 8.4-8.2(brs,2.8,NH⁺) 7.97(s,1,purine H-8), 6.63(br s,2,purine NH₂) 6.16 and 6.00(both m,2,CH=CH), 5.42(m,1,H-4 of cyclopentene), 4.3-4.0(m,3,CH₂OH and CH-O), 3.93(m,1,CH-N of threonyl), 3.14(m,1,H-1 of cyclopentene, 2.73(m,1,0.5CH₂); 1.67(m,1,0.5CH₂), 1.20(d,J=6.6,3,CH₃); MS(CI) : 349(M+1).

| | | | |
|---|---|---|---|
| Anal. Calc. for C₁₅H₂₀N₆O₄^{·}H₂O^{·}0.2EtOH^{·}2.8CF₃CO₂H: | C,36.30; | H,3.77; | N,12.09 |
| Found : | C,36.36; | H,3.95; | N,12.01. |

### Example 2

### Tablet Formulations

The following formulations A, B and C are prepared by wet granulation of the ingredients with a solution of povidone, followed by addition of magnesium stearate and compression.

| Formulation A | | |
|---|---|---|
| | mg/tablet | mg/tablet |
| Active ingredient | 250 | 250 |
| Lactose B.P. | 210 | 26 |
| Povidone B.P. | 15 | 9 |
| Sodium Starch Glycollate | 20 | 12 |
| Magnesium Stearate | 5 | 3 |
| | 5̅0̅0̅ | 3̅0̅0̅ |

| Formulation B | | |
|---|---|---|
| | mg/tablet | mg/tablet |
| Active ingredient | 250 | 250 |
| Lactose | 150 | - |
| Avicel PH 101 | 60 | 26 |
| Povidone B.P. | 15 | 9 |
| Sodium Starch Glycollate | 20 | 12 |
| Magnesium Stearate | 5 | 3 |
| | 5̅0̅0̅ | 3̅0̅0̅ |

| Formulation C | |
|---|---|
| | mg/tablet |
| Active ingredient | 100 |
| Lactose | 200 |
| Starch | 50 |
| Povidone B.P. | 5 |
| Magnesium stearate | 4 |
| | 3̅5̅9̅ |

The following formulations, D and E, are prepared by direct compression of the admixed ingredients. The lactose in formulation E is of the compression type.

| Formulation D | |
|---|---|
| | mg/tablet |
| Active Ingredient | 250 |
| Avicel | 150 |
| Magnesium Stearate | 4 |
| | 4̅0̅4̅ |

| Formulation E | |
|---|---|
| | mg/tablet |
| Active Ingredient | 250 |
| Lactose | 150 |
| Avicel | 100 |
| Magnesium Stearate | 5 |
| | 5̅0̅5̅ |

### Formulation F (Controlled Release Formulation)

The formulation is prepared by wet granulation of the ingredients (below) with a solution of povidone followed by the addition of magnesium stearate and compression.

| | mg/tablet |
|---|---|
| Active Ingredient | 500 |
| Hydroxypropylmethylcellulose (Methocel K4M Premium) | 112 |
| Lactose B.P. | 53 |
| Povidone B.P. | 28 |
| Magnesium Stearate | 7 |
| | 7̅0̅0̅ |

### Example 3

### Capsule Formulations

### Formulation A

A capsule formulation is prepared by admixing the ingredients of Formulation D in Example 2 above and filling into a two-part hard gelatin capsule. Formulation B (infra) is prepared in a similar manner.

| Formulation B | |
|---|---|
| | mg/capsule |
| Active ingredient | 250 |
| Lactose B.P. | 143 |
| Sodium Starch Glycollate | 25 |
| Magnesium Stearate | 2 |
| | 4̅2̅0̅ |

| Formulation C | |
|---|---|
| | mg/capsule |
| Active ingredient | 250 |
| Macrogol 4000 B.P. | 350 |
| | 6̅0̅0̅ |

Capsules are prepared by melting the Macrogol 4000 BP, dispersing the active ingredient in the melt and filling the melt into a two-part hard gelatin capsule.

| Formulation D | |
|---|---|
| | mg/capsule |
| Active ingredient | 250 |
| Lecithin | 100 |
| Arachis Oil | 100 |
| | 4̅5̅0̅ |

Capsules are prepared by dispersing the active ingredient in the lecithin and arachis oil and filling the dispersion into soft, elastic gelatin capsules.

### Formulation E (Controlled Release Capsule)

The following controlled release capsule formulation is prepared by extruding ingredients a), b) and c) using an extruder, followed by spheronisation of the extrudate and drying. The dried pellets are then coated with release-controlling membrane d) and filled into a two-piece, hard gelatin capsule.

| | mg/capsule |
|---|---|
| a) Active Ingredient | 250 |
| b) Microcrystalline Cellulose | 125 |
| c) Lactose B.P. | 125 |
| d) Ethyl Cellulose | 13 |
| | 5̅1̅3̅ |

### Example 4

| Ophthalmic Solution | |
|---|---|
| Active ingredient | 0.5 |
| Propylene Glycol | 0.2 g |
| Thiomersal | 0.001 g |
| Purified water to | 100 ml |
| pH adjusted to | 7.5 |

### Example 5

| Injectable Formulation | |
|---|---|
| Active Ingredient | 0.200 g |
| Sterile, pyrogen free citrate buffer (pH 7.0) to | 10 ml |

The active ingredient is dissolved in most of the citrate buffer (35°-40°C), then made up to volume and filtered through a sterile micropore filter into a sterile 10ml amber glass vial (type 1) and sealed with sterile closures and overseals.

### Example 6

| Intramuscular injection | |
|---|---|
| Active Ingredient | 0.20 g |
| Benzyl Alcohol | 0.10 g |
| Glycofurol 75 | 1.45 g |
| Water for Injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml amber glass vials (type 1).

### Example 7

| Syrup Suspension | |
|---|---|
| Active Ingredient | 0.25 g |
| Sorbitol Solution | 1.50 g |
| Glycerol | 2.00 g |
| Sodium Benzoate | 0.005 g |
| Flavour, | 0.0125 ml |
| Purified Water q.s. to | 5.00 ml |

The sodium benzoate is dissolved in a portion of the purified water and the sorbitol solution added. The active ingredient is added and dissolved. The glycerol and flavours are added and mixed in. Water is added to a final volume of 5ml.

### Example 8

| Suppository | |
|---|---|
| | mg/suppository |
| Active Ingredient | 250 |
| Hard Fat, BP (Witepsol H15 - Dynamit Nobel) | 1700 |
| | 1̅9̅5̅0̅ |

One-fifth of the Witepsol H15 is melted in a steam-jacketed pan at 45°C maximum. The active ingredient is sifted through a 200µm sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250µm stainless steel screen and, with continuous stirring, is allowed to cool to 40°C. At a temperature of 38°C to 40°C, 2.02g of the mixture is filled into suitable plastic moulds. The suppositories are allowed to cool to room temperature.

### Example 9

| Pessaries | |
|---|---|
| | mg/pessary |
| Active ingredient 63µm | 250 |
| Anhydrous Dextrose | 543 |
| Starch | 200 |
| Magnesium Stearate | 7 |
| | 1̅0̅0̅0̅ |

The above ingredients are mixed directly and pessaries prepared by direct compression of the resulting mixture.

### Example 10

### Determination of Oral Bioavailability

The absorption and metabolic disposition of the title compound of Example 1F and its L-valine ester (Example 1G) were studied in male CD rats given single oral doses equivalent to 10mg carbovir per kg body weight. The rats were housed individually in metabolic cages; urines were collected during the 0-24 and 24-48 hour periods post dose and were then filtered and analysed by high-performance liquid chromatography.

| Compound Dosed | % of Dose Recovered as Title compound of Example 1F in Urine |
|---|---|
| Title compound of Example 1F | 12.8 (± 3.4) |
| Title compound of Example 1G | 34.8 (± 1.8) |

After oral doses of the title compound of Example 1G equivalent to carbovir doses of 10 mg/kg, the mean urinary recovery of the title compound of Example 1F accounted for 34.8% of the dose. No unchanged ester was detected in any of the urines. Thus, the valyl ester provides an approximate 3-fold increase in the oral bioavailability of active drug, as compared to dosing with the parent compound itself.

## Claims (Claims for the following Contracting State(s): AT, BE, CH, DE, FR, GB, IT, LI, LU, NL, SE)

1. Compounds of formula (I): 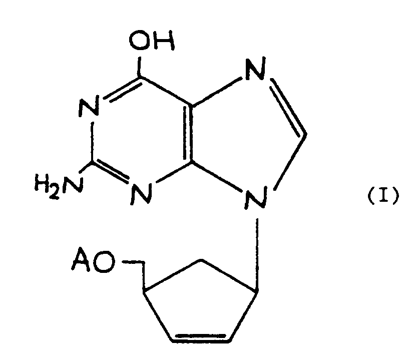 (wherein A is the acyl residue of an aliphatic amino acid containing up to 7 carbon atoms) and pharmaceutically acceptable salts thereof.

2. Compounds of formula (I) as claimed in claim 1 wherein A is the acyl residue of valine, isoleucine, leucine or threonine, and pharmaceutically acceptable salts thereof.

3. A compound of formula (I) as claimed in claim 1) which is (1S,4R)-[4-(2-Amino-1,6-dihydro-6-oxo-9H-purin-9-yl)-2-cyclopenten-1-yl]methyl-L-valinate.

4. Compounds of formula (I) (as defined in claim 1) and pharmaceutically acceptable salts thereof for use in medical therapy.

5. Compounds of formula (I) (as defined in claim 1) and pharmaceutically acceptable salts thereof for use in the treatment or prophylaxis of a viral disease in an animal.

6. Compounds of formula (I) and pharmaceutically acceptable salts thereof as claimed in claim 6 wherein the viral desease is a Human Immunodeficiency Virus (HIV) or Hepatitis B Virus(HBV) infection.

7. Use of compounds of formula (I) (as defined in claim 1) and pharmaceutically acceptable salts thereof in the manufacture of a medicament for the treatment of a viral disease in an animal.

8. Pharmaceutical formulations comprising at least one compound of formula (I) (as defined in claim 1) or a pharmaceutically acceptable salt thereof together with one or more pharmaceutical carriers therefor.

9. A process for the preparation of compounds of formula (I) (as defined in claim 1) and pharmaceutically acceptable salts thereof which comprises either:-
(a) reacting a compound of formula (II) 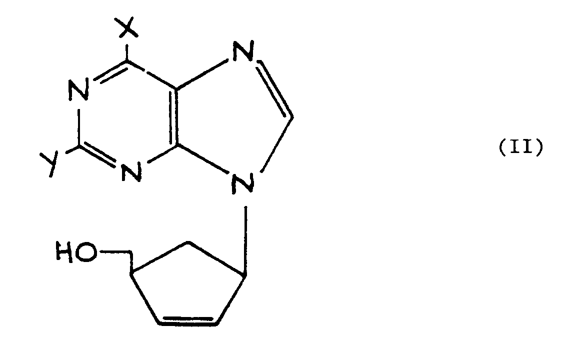 wherein X is an optionally protected hydroxy group, and Y is an optionally protected amino group, with an optionally protected aliphatic amino acid containing up to 7 carbon atoms or a functional equivalent thereof to introduce the acyl residue of an amino acid at the 5'-position; or
(b) converting a compound of formula (III) 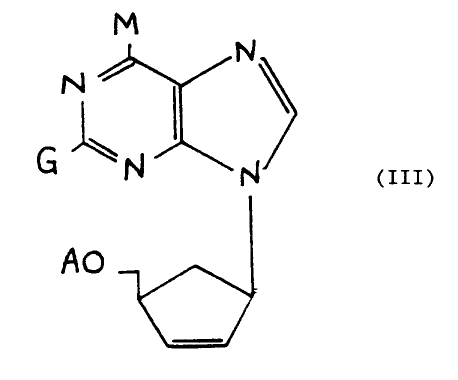 wherein A is as defined in claim 1; and M represents a hydroxy group and G represents an atom or group that can be replaced by or converted to an amino group; or G represents an amino group and M represents an atom or group that can be replaced by or converted to a hydroxy group, into a compound of formula (I) or a pharmaceutically acceptable derivative thereof; or
(c) reacting a compound of formula (IV) 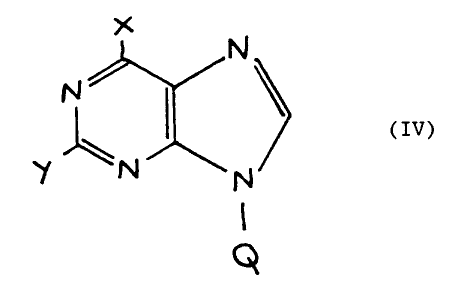 (wherein X and Y are as defined above and Q represents a leaving atom or group) with a compound of formula (V) 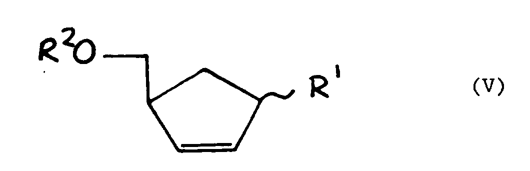 wherein R¹ represents a leaving group or atom and R² represents an optionally protected acyl residue of an aliphatic amino acid containing up to 7 carbon atoms; and optionally effecting one or more of the following conversions in any desired sequence:-
i) removal of any protecting groups;
ii) where the resulting product is a compound of formula (I). conversion of the said compound into a pharmaceutically acceptable salt thereof; and
iii) where the resulting product is a pharmaceutically acceptable salt of a compound of formula (I), conversion of the said salt into the parent compound.

## Claims (Claims for the following Contracting State(s): ES, GR)

1. A process for the preparation of compounds of formula (I): 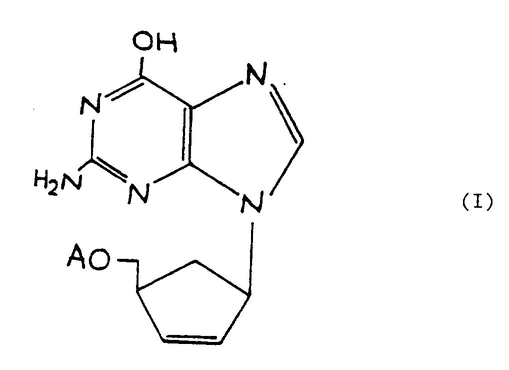 (wherein A is the acyl residue of an aliphatic amino acid containing up to 7 carbon atoms) and pharmaceutically acceptable salts thereof, which comprises either:-
(a) reacting a compound of formula (II) 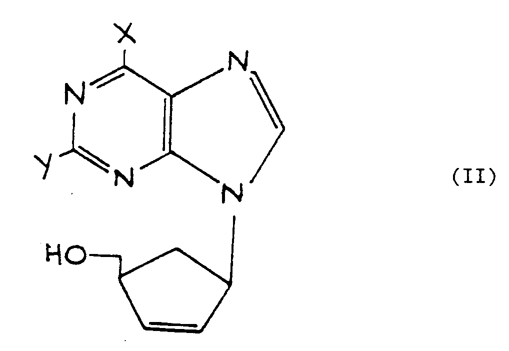 wherein X is an optionally protected hydroxy group, and Y is an optionally protected amino group, with an optionally protected aliphatic amino acid containing up to 7 carbon atoms or a functional equivalent thereof to introduce the acyl residue of an amino acid at the 5'-position; or
(b) converting a compound of formula (III) 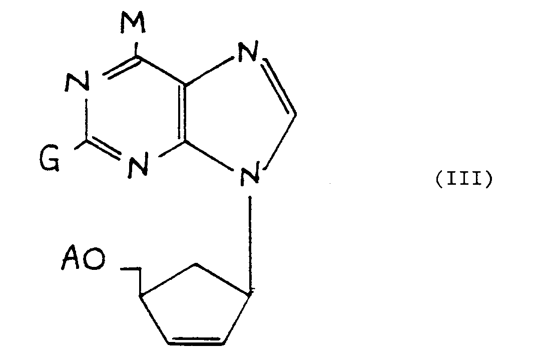 wherein A is as defined above; and M represents a hydroxy group and G represents an atom or group that can be replaced by or converted to an amino group; or G represents an amino group and M represents an atom or group that can be replaced by or converted to a hydroxy group, into a compound of formula (I) or a pharmaceutically acceptable derivative thereof; or
(c) reacting a compound of formula (IV) 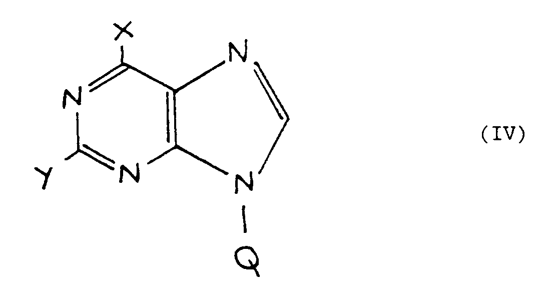 (wherein X and Y are as defined above and Q represents a leaving atom or group) with a compound of formula (V) 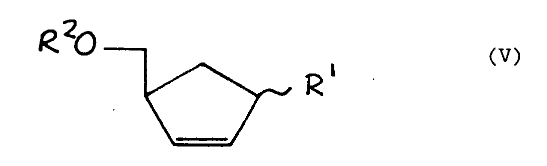 wherein R¹ represents a leaving group or atom and R² represents an optionally protected acyl residue of an aliphatic amino acid containing up to 7 carbon atoms; and optionally effecting one or more of the following conversions in any desired sequence:-
i) removal of any protecting groups;
ii) where the resulting product is a compound of formula (I). conversion of the said compound into a pharmaceutically acceptable salt thereof; and
iii) where the resulting product is a pharmaceutically acceptable salt of a compound of formula (I), conversion of the said salt into the parent compound.

2. A process as claimed in claim 1 wherein A is the acyl residue of valine, isoleucine, t-butylglycine or threonine, and pharmaceutically acceptable salts thereof.

3. A process as claimed in claim 1 for the preparation of (1S,4R)-[4-(2-Amino-1,6-dihydro-6-oxo-9H-purin-9-yl)-2-cyclopenten-1-yl]methyl-L-valinate.

## Patentansprüche (Patentansprüche für folgende(n) Vertragsstaat(en): AT, BE, CH, DE, FR, GB, IT, LI, LU, NL, SE)

1. Verbindungen der Formel (I): 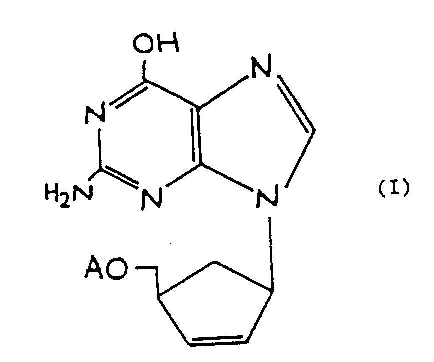 (worin A der Acylrest einer aliphatischen Aminosäure mit bis zu 7 Kohlenstoffatomen ist) und pharmazeutisch annehmbare Salze davon.

2. Verbindungen der Formel (I) gemäß Anspruch 1, worin A der Acylrest von Valin, Isoleucin, Leucin oder Threonin ist, und pharmazeutisch annehmbare Salze davon.

3. Verbindung der Formel (I) nach Anspruch 1, nämlich (1S,4R)-[4-(2-Amino-1,6-dihydro-6-oxo-9H-purin-9-yl)-2-cyclopenten-1-yl]-methyl-L-valinat.

4. Verbindungen der Formel (I) (wie definiert in Anspruch 1) und pharmazeutisch annehmbare Salze davon zur Verwendung bei der medizinischen Behandlung.

5. Verbindung der Formel (I) (wie definiert in Anspruch 1) und pharmazeutisch annehmbare Salze davon zur Verwendung bei der Behandlung oder Prophylaxe einer Viruserkrankung bei einem Tier.

6. Verbindungen der Formel (I) und pharmazeutisch annehmbare Salze davon gemäß Anspruch 6, worin die virale Erkrankung eine human Immunschwächevirus (HIV)- oder Hepatitis B Virus (HBV)-Infektion ist.

7. Verwendung der Verbindungen der Formel (I) (wie definiert in Anspruch 1) und pharmazeutisch annehmbare Salze davon bei der Herstellung eines Medikaments zur Behandlung einer viralen Erkrankung bei einem Tier.

8. Pharmazeutische Formulierungen, enthaltend mindestens eine Verbindung der Formel (I) (wie in Anspruch 1 definiert) oder ein pharmazeutisch annehmbares Salz davon zusammen mit einem oder mehreren pharmazeutischen Trägern dafür.

9. Verfahren zur Herstellung einer Verbindung der Formel (I) (wie definiert in Anspruch 1) und pharmazeutisch annehmbare Salze davon, welches umfaßt entweder:
(a) Umsetzen einer Verbindung der Formel (II) 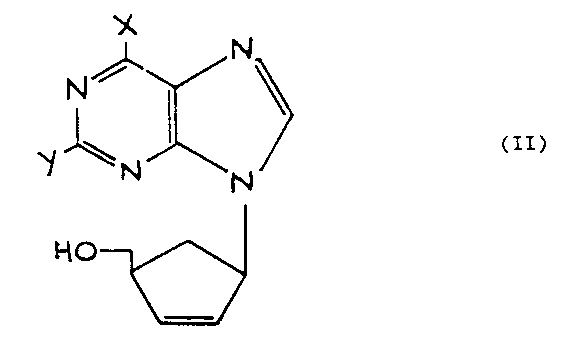 worin X eine wahlweise geschützte Hydroxylgruppe und Y eine wahlweise geschützte Aminogruppe ist, mit einer wahlweise geschützten aliphatischen Aminosäure mit bis zu 7 Kohlenstoffatomen oder einem funktionellen Äquivalent davon unter Einführung des Acylrests einer Aminosäure in die 5'-Position, oder
(b) Umwandeln einer Verbindung der Formel (III) 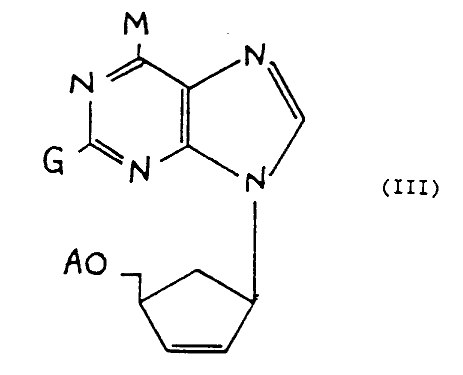 worin A wie in Anspruch 1 definiert ist und M eine Hydroxylgruppe und G ein Atom oder eine Gruppe darstellt, die durch eine Aminogruppe ersetzt oder in eine solche umgewandelt werden kann, oder G stellt eine Aminogruppe dar und M stellt ein Atom oder eine Gruppe dar, die durch eine Hydroxylgruppe ersetzt oder in eine solche umgewandelt werden kann, in eine Verbindung der Formel (I) oder ein pharmazeutisch annehmbares Derivat davon, oder
(c) Umsetzen einer Verbindung der Formel (IV) 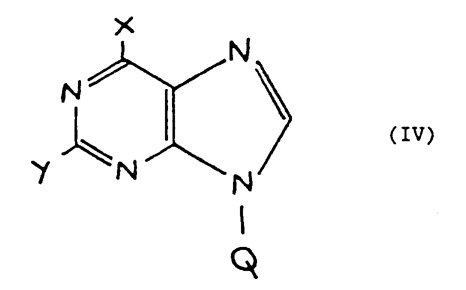 (worin X und Y dieselben Bedeutungen wie oben haben und Q ein Austrittsatom oder eine Austrittsgruppe darstellt) mit einer Verbindung der Formel (V) 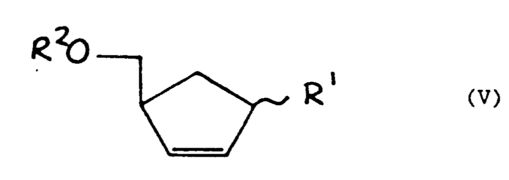 worin R¹ eine Austrittsgruppe oder -atom und R² einen wahlweise geschützten Acylrest einer aliphatischen Aminosäure mit bis zu 7 Kohlenstoffatomen darstellt, und wahlweises Durchführen einer oder mehrerer der folgenden Umwandlungen in beliebiger Reihenfolge:
i) Entfernen der Schutzgruppen;
ii) wenn das erhaltene Produkt eine Verbindung der Formel (I) ist, Umwandlung der Verbindung in ein pharmazeutisch annehmbares Salz davon; und
iii) wenn das erhaltene Produkt ein pharmazeutisch annehmbares Salz einer Verbindung der Formel (I) ist, Umwandeln des Salzes in die Stammverbindung.

## Patentansprüche (Patentansprüche für folgende(n) Vertragsstaat(en): ES, GR)

1. Verfahren zur Herstellung einer Verbindung der Formel (I): 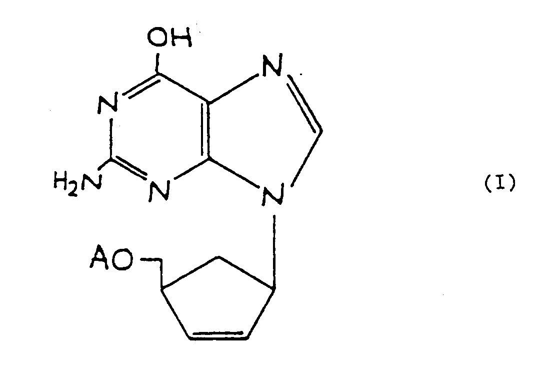 (worin A der Acylrest einer aliphatischen Aminosäure mit bis zu 7 Kohlenstoffatomen ist) und pharmazeutisch annehmbare Salze davon, welches umfaßt entweder:
(a) Umsetzen einer Verbindung der Formel (II) 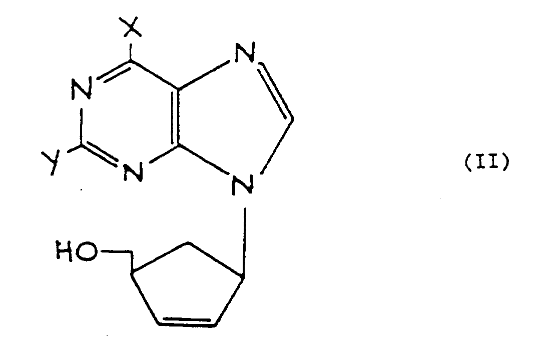 worin X eine wahlweise geschützte Hydroxylgruppe und Y eine wahlweise geschützte Aminogruppe ist, mit einer wahlweise geschützten aliphatischen Aminosäure mit bis zu 7 Kohlenstoffatomen oder einem funktionellen Äquivalent davon zur Einführung des Acylrests einer Aminosäure in die 5'-Position, oder
(b) Umwandeln einer Verbindung der Formel (III) 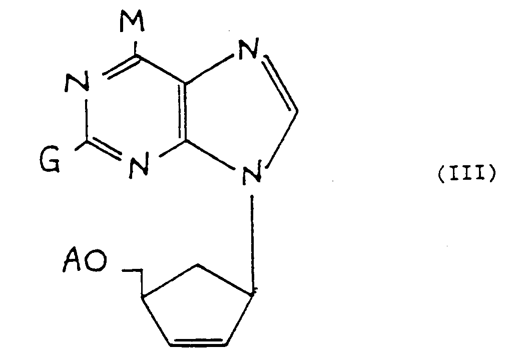 worin A wie in Anspruch 1 definiert ist und M eine Hydroxylgruppe und G ein Atom oder eine Gruppe darstellt, die durch eine Aminogruppe ersetzt oder in eine solche umgewandelt werden kann, oder G stellt eine Aminogruppe dar und M stellt ein Atom oder eine Gruppe dar, die durch eine Hydroxylgruppe ersetzt oder in eine solche umgewandelt werden kann, in eine Verbindung der Formel (I) oder ein pharmazeutisch annehmbares Derivat davon, oder
(c) Umsetzen einer Verbindung der Formel (IV) 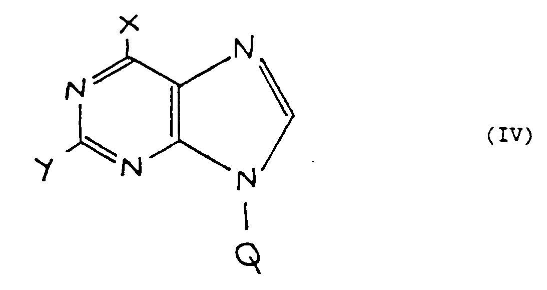 (worin X und Y dieselben Bedeutungen wie oben haben und Q ein Austrittsatom oder eine Austrittsgruppe darstellt) mit einer Verbindung der Formel (V) 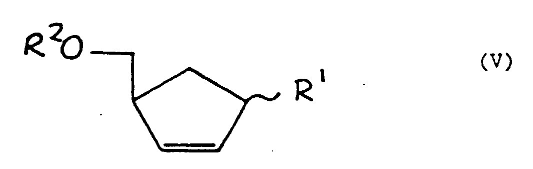 worin R¹ eine Austrittsgruppe oder ein Austrittsatom und R² einen wahlweise geschützten Acylrest einer aliphatischen Aminosäure mit bis zu 7 Kohlenstoffatomen darstellt, und wahlweises Durchführen einer oder mehrerer der folgenden Unwandlungen in beliebiger Reihenfolge:
i) Entfernen der Schutzgruppen;
ii) wenn das erhaltene Produkt eine Verbindung der Formel (I) ist, Umwandlung der Verbindung in ein pharmazeutisch annehmbares Salz davon; und
iii) wenn das erhaltene Produkt ein pharmazeutisch annehmbares Salz einer Verbindung der Formel (I) ist, Umwandeln des Salzes in die Stammverbindung.

2. Verfahren nach Anspruch 1, worin A der Acylrest von Valin, Isoleucin, t-Butylglycin oder Threonin ist und pharmazeutisch annehmbare Salze davon.

3. Verfahren nach Anspruch 1 zur Herstellung von (1S,4R)-[4-(2-Amino-1,6-dihydro-6-oxo-9H-purin-9-yl)-2-cyclopenten-1-yl]-methyl-L-valinat.

## Revendications (Revendications pour l'(les) Etat(s) contractant(s) suivant(s): AT, BE, CH, DE, FR, GB, IT, LI, LU, NL, SE)

1. Composés de formule (I) : 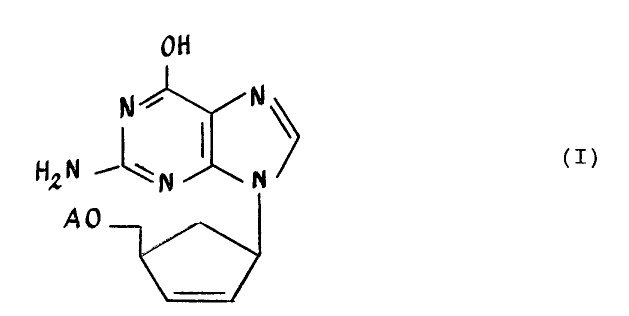 (où A est le reste acyle d'un acide aminé aliphatique comptant jusqu'à 7 atomes de carbone) et leurs sels pharmaceutiquement acceptables.

2. Composés de formule (I) suivant la revendication 1, où A est le reste acyle de la valine, de l'isoleucine, de la leucine ou de la thréonine, et leurs sels pharmaceutiquement acceptables.

3. Composé suivant la revendication 1, qui est le L-valinate de (1S,4R)-[4-(2-amino-1,6-dihydro-6-oxo-9H-purin-9-yl)-2-cyclopentén-1-yl]méthyle.

4. Composés de formule (I) (tels que définis dans la revendication 1) et leurs sels pharmaceutiquement acceptables à utiliser en thérapeutique médicale.

5. Composés de formule (I) (tels que définis dans la revendication 1) et leurs sels pharmaceutiquement acceptables à utiliser dans le traitement ou la prophylaxie d'une maladie virale chez un animal.

6. Composés de formule (I) et leurs sels pharmaceutiquement acceptables suivant la revendication 5, dans lesquels la maladie virale est une infection par le virus de l'immunodéficience humaine (VIH) ou le virus de l'hépatite B (VHB).

7. Utilisation de composés de formule (I) (tels que définis dans la revendication 1) et de leurs sels pharmaceutiquement acceptables dans la fabrication d'un médicament pour le traitement d'une affection virale chez un animal.

8. Compositions pharmaceutiques comprenant au moins un composé de formule (I) (tel que défini dans la revendication 1) ou un sel pharmaceutiquement acceptable de celui-ci conjointement avec un ou plusieurs excipients pharmaceutiquement acceptables.

9. Procédé de préparation de composés de formule (I) (tels que définis dans la revendication 1) et de leurs sels pharmaceutiquement acceptables, qui comprend :
a) la réaction d'un composé de formule (II) : 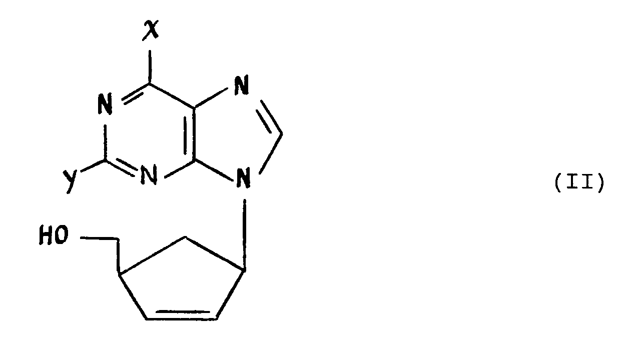 où X est un radical hydroxyle facultativement protégé et Y est un radical amino facultativement protégé, avec un acide aminé aliphatique facultativement protégé, comptant jusqu'à 7 atomes de carbone, ou un dérivé fonctionnel de celui-ci, pour introduire le reste acyle d'un acide aminé à la position 5', ou
b) la conversion d'un composé de formule (III) : 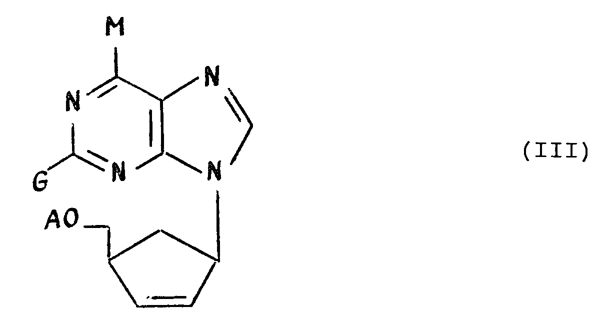 où A est tel que défini dans la revendication 1, M représente un radical hydroxyle et G représente un atome ou radical qui peut être remplacé par ou converti en un radical amino; ou G représente un radical amino et M représente un atome ou radical qui peut être remplacé par ou converti en un radical hydroxyle, en un composé de formule (I) ou en un dérivé pharmaceutiquement acceptable de celui-ci, ou
c) la réaction d'un composé de formule (IV) : 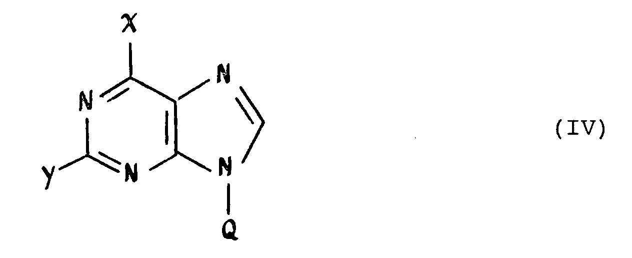 (où X et Y sont tels que définis ci-dessus et Q représente un atome ou radical partant) avec un composé de formule (V) : 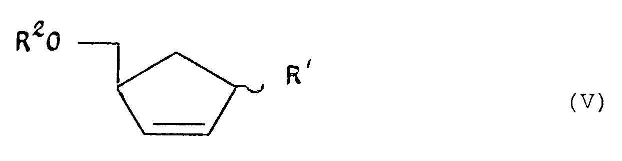 où R¹ représente un atome ou radical partant et R² représente un reste acyle facultativement protégé d'un acide aminé aliphatique comptant jusqu'à 7 atomes de carbone, et facultativement l'exécution d'une ou plusieurs des conversions suivantes dans tout ordre souhaité :
i) l'élimination de radicaux protecteurs éventuels;
ii) lorsque le produit résultant est un composé de formule (I), la conversion de ce composé en un sel pharmaceutiquement acceptable de celui-ci, et
iii) lorsque le produit résultant est un sel pharmaceutiquement acceptable d'un composé de formule (I), la conversion de ce sel en le composé parent.

## Revendications (Revendications pour l'(les) Etat(s) contractant(s) suivant(s): ES, GR)

1. procédé de préparation de composés de formule (I) : 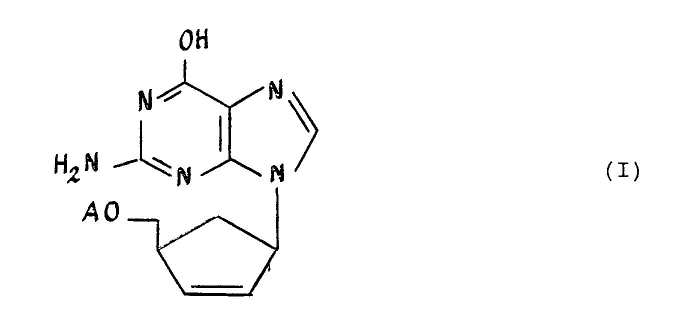 (où A est le reste acyle d'un acide aminé aliphatique comptant jusqu'à 7 atomes de carbone) et de leurs sels pharmaceutiquement acceptables, qui comprend :
a) la réaction d'un composé de formule (II) : 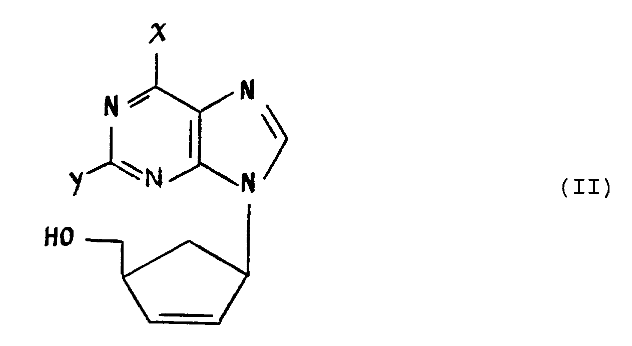 où X est un radical hydroxyle facultativement protégé et Y est un radical amino facultativement protégé, avec un acide aminé aliphatique facultativement protégé, comptant jusqu'à 7 atomes de carbone, ou un dérivé fonctionnel de celui-ci, pour introduire le reste acyle d'un acide aminé à la position 5', ou
b) la conversion d'un composé de formule (III) : 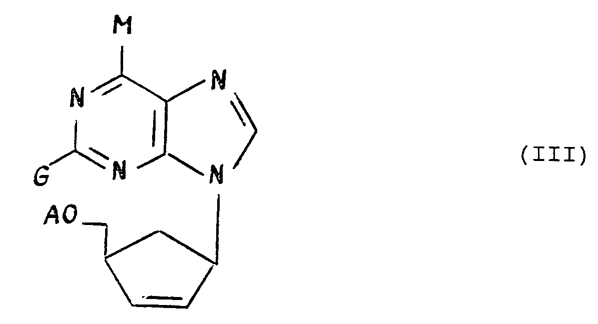 où A est tel que défini ci-dessus, M représente un radical hydroxyle et G représente un atome ou radical qui peut être remplacé par ou converti en un radical amino; ou G représente un radical amino et M représente un atome ou radical qui peut être remplacé par ou converti en un radical hydroxyle, en un composé de formule (I) ou en un dérivé pharmaceutiquement acceptable de celui-ci, ou
c) la réaction d'un composé de formule (IV) : 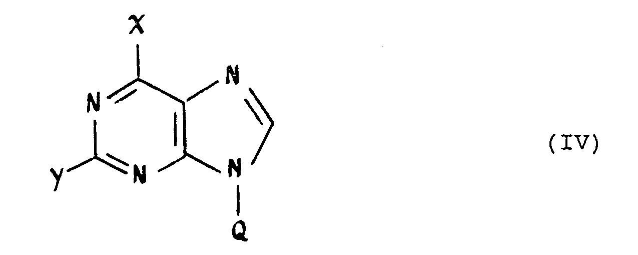 (où X et Y sont tels que définis ci-dessus et Q représente un atome ou radical partant) avec un composé de formule (V) : 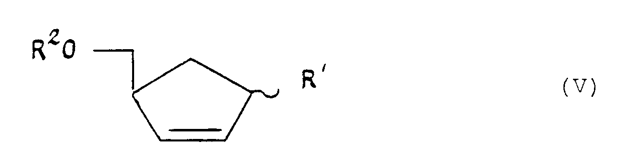 où R¹ représente un atome ou radical partant et R² représente un reste acyle facultativement protégé d'un acide aminé aliphatique comptant jusqu'à 7 atomes de carbone, et facultativement l'exécution d'une ou plusieurs des conversions suivantes dans tout ordre souhaité :
i) l'élimination de radicaux protecteurs éventuels;
ii) lorsque le produit résultant est un composé de formule (I), la conversion de ce composé en un sel pharmaceutiquement acceptable de celui-ci, et
iii) lorsque le produit résultant est un sel pharmaceutiquement acceptable d'un composé de formule (I), la conversion de ce sel en le composé parent.

2. Procédé suivant la revendication 1, dans lequel A est le reste acyle de la valine, de l'isoleucine, de la t-butylglycine ou de la thréonine, et leurs sels pharmaceutiquement acceptables.

3. Procédé suivant la revendication 1, de préparation du L-valinate de (1S,4R)-[4-(2-amino-1,6-dihydro-6-oxo-9H-purin-9-yl)-2-cyclopentén-1-yl]méthyle.
